# EUROPEAN PATENT APPLICATION

(11) **EP 3 385 340 A1**
(43) Date of publication of application: **10.10.2018**
(21) Application number: 17165468.4
(22) Date of filing: 07.04.2017
(51) Int. Cl.: C09D 5/16, A61L 29/04, A61L 29/08, A61L 29/14, A61L 31/04, A61L 31/10, A61L 31/14, C09D 105/00, C09D 105/04, C09D 105/08, A61L 27/20, A61L 27/34, A61L 27/50

(54) **COATING FOR IMPLANTABLE MEDICAL DEVICE**

(71) Applicant: NovioSense B.V., 6534 AT Nijmegen (NL)
(72) Inventor: The designation of the inventor has not yet been filed
(74) Representative: Ishiguro, Masaoki

(57) **Abstract**

The invention relates to a coating comprising a hydrogel layer of a hydrogel of a polysaccharide and polymer brushes attached to the hydrogel layer. The invention also relates to a process for making the coating according to any one of the preceding claims, comprising the steps of: a) providing the hydrogel layer on a substrate, b) attaching an initiator having a radically transferable atom on the hydrogel layer and c) reacting monomers with the initiator to form the polymer brushes attached to the hydrogel layer.

## Description

The present invention relates to a coating for an implantable medical device and an implantable medical device provided with such coating.

Continuous health monitoring using wearable sensors linked to cell phones is rapidly becoming a reality. For several decades, developing implantable biosensors has been a major focus of research, especially for non-communicable diseases like diabetes. Being able to monitor glucose subcutaneously over long periods would result in higher compliance and thus lower the cost of treatment. A biocompatible implant should ideally integrate and interface with surrounding tissue, facilitate the measurement of the target analyte and maintain the required accuracy time over its lifetime. Upon implantation of a foreign device inside of the human body, its surface suffers the rapid adsorption of proteins (fouling), eliciting cellular and tissue responses that encapsulate the foreign body and ultimately diverge from natural wound healing process. The formation of a granulomar around a foreign body results in impaired fluid transport to the sensor site, leading to a significant change in sensor response over time.

Use of antifouling polymer brushes for preventing the adsorption of proteins to the surface is known. For example, Biomacromolecules 2016, 17, 1179-1185 describes providing a polycarbonate surface with antifouling polymer brushes made of polymers such as poly(HEMA). In this document, the polycarbonate surface was silanized and polymer brushes were grafted by atom transfer radical polymerization (ATRP). Polym. Chem., 2016, 7, 6934-6945 describes photoinduced single-electron transfer living radical polymerization (SET-LRP) for the preparation of polymer brushes on silicon wafer chips from a variety of methacrylate monomers. The process consists of forming a self-assembled monolayer (SAM) of the initiator on the silicon substrate and photoinduced polymerization of the monomer.

Macromol. Biosci. 2015, 15, 6346-646 describes preparation of polymer brushes by coating gold on Si-wafer chips followed by surface-initiated atom transfer radical polymerization (SI-ATRP).

These known methods have a disadvantage for the application on medical devices in that the resulting modified surfaces may not be suitable for all situations and requires the medical device to be subjected again to time consuming compatibility tests. Further, the necessity for chemical modification (silanization or formation of SAM) may limit the type of the substrate to which these methods can be applied

It is an objective of the invention to solve the above-mentioned and/or other problems.

The invention provides a coating comprising a hydrogel layer of a hydrogel of a polysaccharide and polymer brushes attached to the hydrogel layer. The polymer brush is attached on one end to the surface of the hydrogel layer and comprises repeating units with a side chain which provides the surface with protein repelling properties. The polymer brushes prevent protein adhesion to the hydrogel layer which results in the anti-fouling property.

Advantageously, a polysaccharide hydrogel can be attached to a wide range of surfaces with sufficient strength, without modification of the surface. A layer of a polysaccharide hydrogel can be formed by crosslinking polysaccharide on the surface. Polysaccharide is a high molecular weight natural polymer only soluble at low pH in aqueous conditions. As the molecular weight of the chains increases due to crosslinking, it becomes more insoluble. The deposition of the non-covalently bound film on the surface results from increased insolubility of the high molecular weight cross-linked chains and the non-covalent interactions occurring at the interface between the hydrogel and the substrate surface. Since no reaction takes place between the polysaccharide and the surface, the substrate material can be of a wide range of type.

The inventors have noted that a polysaccharide hydrogel coating without polymer brushes is not sufficient for a medical device to be implanted. Although a polysaccharide in itself exhibits a low toxicity, when a polysaccharide is coated onto a surface and combined with the formation of a wound on implantation, protein-polysaccharide interactions have been shown to occur leading to an inflammation response and ultimately encapsulation of the coated device.

According to the invention, the polysaccharide hydrogel layer is grafted with polymer brushes which have anti-fouling effect. Accordingly, the coating according to the invention can be used as a coating for an implantable medical device.

According to another aspect, the present invention provides a process for making the coating according to the invention, comprising the steps of:
a) providing the hydrogel layer on a substrate,
b) attaching an initiator having a radically transferable atom on the hydrogel layer and
c) reacting monomers with the initiator to form the polymer brushes attached to the hydrogel layer.

### Step a)

Preferably, the polysaccharide is selected from the group consisting of alginate, chitosan, hyaluronan and chondroitin sulfate, particularly preferably chitosan.

The hydrogel layer may be provided on a substrate of various types. For example, the substrate may be made of a material selected from the group consisting of silicon, glass, stainless steel, platinum. platinum iridium, nitinol, tantalum, titanium, cobalt-chromium alloy, copper, silver, copper-silver alloy, silicone, polyethylene, polycarbonate, polyurethane, polycaprolactone, silk and collagen.

Step a) may be performed by any known way for providing a hydrogel layer. Preferably, step a) involves crosslinking the polysaccharide on the substrate to form the hydrogel layer. Typically, the polysaccharide and a crosslinker are provided on the substrate on which the hydrogel layer is to be formed. This may be done e.g. by spin coating, dip coating, spray coating etc. A solution of the polysaccharide and a solution of the crosslinker may be provided separately on the substrate or a solution comprising both the polysaccharide and the crosslinker may be provided on the substrate. Suitable crosslinkers are well-known, for example glutaraldehyde, genipin, dicarboxylic acids, dianhydrides, *N*-hydroxysuccinimide esters, imidoesters, carbodiimides.

Preferably, the solution of the monomers and the solution of the crosslinker are aqueous solutions. This is desirable in view of environmental aspects and ease of the process.

### Step b)

The initiator has an anchoring group which reacts with the functional groups of the polysaccharide. This anchoring group anchors the initiator on the surface of the hydrogel layer. The initiator further has a functional group which acts as an initiating site for the polymerization in step c), which is a group having the radically transferable atom.

The initiator may be represented by a formula having on one end a group having the radically transferable atom X and on another end an anchoring group Y which reacts with the functional groups of the polysaccharide.

Suitable examples of the initiators include compounds represented by formulas 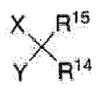 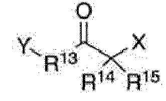 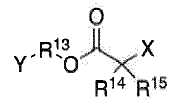 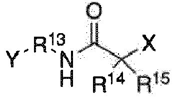 wherein
X is selected from the group consisting of Cl, Br and I;
Y is an anchoring group which reacts with functional groups of the polysaccharide;
R¹³ is selected from the group consisting of C₁-C₂₀ linear or branched alkyl, C₂-C₆ alkenyl, C₂-C₆ alkynyl, N-hydroxysuccinimide, poly(ethylene glycol) methyl ether, C₁₋C₂₀ alkyl substituted with e.g. an azide, and 2,2-dimethyl-1,3-dioxolan-4-yl and
R¹⁴ and R¹⁵ are each independently selected from the group consisting of
   C₁-C₄ linear alkyl, C₂-C₈ cycloalkyl, aryl, heterocyclyl, aralkyl, aralkenyl.

These initiators are particularly suitable when step c) is performed by the living free-radical polymerization, in particular single-electron transfer living radical polymerization (SET-LRP).

Y can be of various types depending on the type of the functional group of the polysaccharide. For example, in the cases where the functional groups of the polysaccharide are -OH or NH2 groups, such as in the case of chitosan, suitable examples of Y include COCl, COBr, COI, CN, oxiranyl, glycidyl and C₂-C₆ alkylene or alkenylene substituted with oxiranyl or glycidyl. In the cases where the functional groups of the polysaccharide are carboxylic groups or sulfonic acid, such as in the case of alginate, hyaluronan and chondroitin sulfate, suitable examples of Y include halogen ions Cl⁻, Br⁻ and I⁻, Grignard reagent, sulfide, azide.

Preferably, Y is COCl or COBr.

Preferably, R¹⁴ is C1-C3 alkyl and/or R¹⁵ is C1-C3 alkyl.

Particularly preferred examples of the initiators include (alpha)bromoisobutyryl bromide (Y-CR¹⁴R¹⁵-X wherein X=Br, Y=COBr, R¹⁴=CH₃, R¹⁵= CH₃).

An initiator may be attached to the hydrogel layer by known methods. For example, a solution containing the initiator may be added dropwise to the layer. The solvent of such solution may be water or an organic solvent, depending on the type of the initiator as can easily be determined by the skilled person. From environmental perspective, it is preferred that the solution containing the initiator is an aqueous solution.

The coating according to the invention should have a relatively dense distribution of the polymer brushes at its surface to have an anti-fouling property. Accordingly, the surface of the hydrogel layer should have a sufficient density of the initiator, from which the polymer brushes can grow. The amount of the initiator to be added to the hydrogel layer can be easily determined by the person skilled in the art to give the final coating sufficient anti-fouling properties. Suitable amount of the initiator to be added to the hydrogel layer may depend on the type of the monomers for forming the polymer brushes. When the monomers have bulkier side branches, the amount of the initiator may be smaller.

### Step c)

In step c), polymerization of the monomers occurs to form polymer brushes. Preferably, step c) is performed by the living free-radical polymerization.

Preferably, the polymer brushes are polymers of a monomer represented by formula (I) or (II) 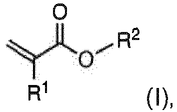 wherein R¹ is H, CH₃ or C₂H₅ and R² is selected from the group consisting of: C1-C12 linear, branched or cyclic alkyl,
- -R⁶-OH wherein R⁶ is C1-C12 linear, branched or cyclic alkyl,
- -R⁷-NH₂ wherein R⁷ is C1-C12 linear, branched or cyclic alkyl; 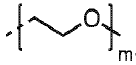 wherein m is 1 to 12, 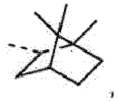 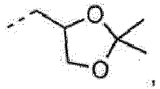 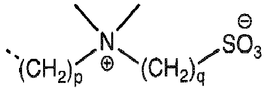 wherein p is 1 to 5 and q is 1 to 5 and 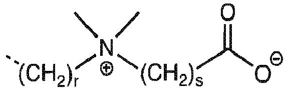 wherein r is 1 to 5 and s is 1 to 5;

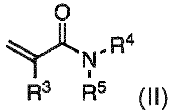

wherein R³ is H, CH₃ or C₂H₅ and
R⁴ and R⁵ are each independently selected from the group consisting of: H, C1-C12 linear, branched or cyclic alkyl;
   - -R⁸-OH wherein R⁸ is C1-C12 linear, branched or cyclic alkyl,
   - -R⁹-NH₂ wherein R⁹ is C1-C12 linear, branched or cyclic alkyl; 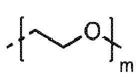 wherein m is 1 to 12, 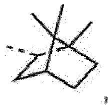 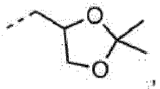 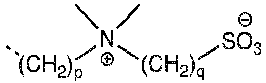 wherein p is 1 to 5 and q is 1 to 5 and 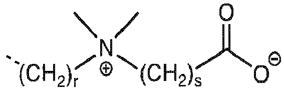 wherein r is 1 to 5 and s is 1 to 5.

It will be appreciated that the above groups described for R2 are bonded to the O atom at the position indicated by a broken line - -. Similarly, it will be appreciated that the above groups described for R³ and R⁴ are bonded to the N atom at the position indicated by - -.

In formula (I):
- R¹: is preferably H or CH₃, and/or
- R²: is preferably oligo (ethylene glycol), 2-hydroxyethyl or 2-hydroxypropyl.

When R² is R⁶-OH, R⁶ is preferably C1-C3 alkyl.

When R² is R⁷-NH2, R⁷ is preferably C1-C3 alkyl.

In formula (II):
- R³: is preferably H or CH₃.

Preferably, one of R⁴ and R⁵ is H and one of R⁴ and R⁵ is R⁸-OH.
- R⁸: is preferably C1-C3 alkyl.

Particularly preferred examples of the monomer are selected from the group consisting of
2-(Dimethylamino)ethyl methacrylate (DMAEMA),
2-hydroxyethyl methacrylate (HEMA),
2-hydroxypropyl methacrylate (HPMA),
oligo(ethylene glycol) methyl ether methacrylate (MeOEGMA),
t-butyl methacrylate (tBMA),
n-butyl methacrylate (nBMA),
methyl methacrylate (MMA),
2-ethylhexyl methacrylate (EHMA),
isobornyl methacrylate (IBMA),
solketal methacrylate (SMA),
N-(2-hydroxypropyl) methacrylamide (HPMAm)
[2-(methacryloyloxy)ethyl]dimethyl-(3-sulfopropyl)ammonium hydroxide (SBMA)
2-Carboxy- N,N-dimethyl-N-(2'-(methacryloyloxy)ethyl)ethanaminium (CBMA).

Direct polymerization from initiator-functionalized surfaces allows achieving very dense polymer brushes and good control over the thickness, composition and architecture.

More bulky monomers result in thicker polymer brushes. This may be preferred when the amount of the initiator attached to the surface of the hydrogel layer is small. With the same number of polymer brushes, a higher density of the polymer brushes can be obtained.

Preferably, the polymer brushes have a Mw of 0.5-500 kDa, for example 1-200 kDa, 5-150 kDa, 10-100 kDa or 20-100 kDa.

Preferably, the number of repeating units in the polymer brushes is in the range of 10-100000, for example 20 - 20000 or 100-10000.

Examples of the living free-radical polymerization are surface initiated atom transfer radical polymerization (SI-ATRP), surface initiated reversible addition-fragmentation chain transfer polymerization (SI-RAFT), surface-initiated nitroxide mediated polymerization (SI-NMP), surface-initiated photoiniferter-mediated polymerization (SI-PIMP) and single-electron transfer living radical polymerization (SET-LRP). These methods are well-known. For example, growing of polymer brushes by SET-LRP is described in detail in Polym. Chem., 2016, 7, 6934-6945.

Preferably, step c) is performed by SET-LRP.

Alternatively step c) is performed by SI-RAFT.

Suitable examples of the initiators used for SI-RAFT include compounds represented by formulas 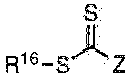 wherein
Z is selected from the group consisting of Ph, SMe, Me, OPh, OEt, N(Et)₂, 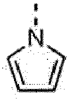 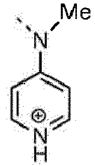 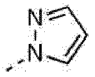 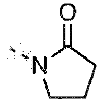 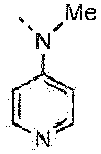 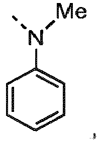
R¹⁶ is selected from the group consisting of 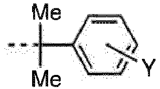 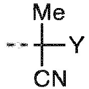 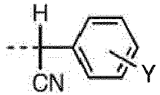 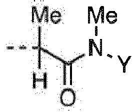 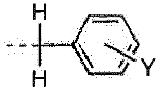 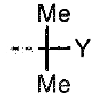 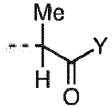 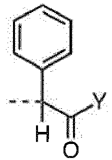 wherein Y is an anchoring group which reacts with functional groups of the polysaccharide.

It will be appreciated that the above groups described for Z are bonded to the C atom at the position indicated by - -. Similarly, it will be appreciated that the above groups described for R¹⁶ are bonded to the S atom at the position indicated by - -.
For the avoidance of doubt, Ph represents phenyl, Me represents methyl and Et represents ethyl.

Y can be of various types depending on the type of the functional group of the polysaccharide. For example, in the cases where the functional groups of the polysaccharide are -OH or NH2 groups, such as in the case of chitosan, suitable examples of Y include COCl, COBr, COI, CN, oxiranyl, glycidyl and C₂-C₆ alkylene or alkenylene substituted with oxiranyl or glycidyl. In the cases where the functional groups of the polysaccharide are carboxylic groups or sulfonic acid, such as in the case of alginate, hyaluronan and chondroitin sulfate, suitable examples of Y include halogen ions X-,Grignard RMgBr, sulfide, azide.

Z can be chosen depending on the chosen monomer in order to provide control and high chain end fidelity. Preferably Z is selected from the group consisting of Ph, SMe, Me and OPh.

Suitable initiators for other types of the living free-radical polymerization such as SI-ATRP, SI-NMP and SI-PIMP can be found in the literature and can suitably be selected by the skilled person.

According to another aspect, the present invention provides a medical device provided with the coating according to the invention. In particular, the present invention provides a medical device encapsulated in the coating according to the invention.

Preferred examples of the medical device include an implantable biosensor, a feeding tube, a central line catheter, a nephrostemy catheter, a guiding catheter, a pacemaker, an internal insulin pump, an orthopedic hip implant, an orthopedic screw, a dental implant, a percutaneous fixation device, a peripherally inserted central catheter, a drainage catheter and a stent such as coronary stent, vascular stent, ureteral stent, prostatic tent, esophageal stent and biliary stent.

According to another aspect, the present invention provides use of the coating according to the invention for encapsulating a medical device, in particular an implantable biosensor.

It is noted that the invention relates to all possible combinations of features described herein, preferred in particular are those combinations of features that are present in the claims. It will therefore be appreciated that all combinations of features relating to the composition according to the invention; all combinations of features relating to the process according to the invention and all combinations of features relating to the composition according to the invention and features relating to the process according to the invention are described herein.

It is further noted that the term 'comprising' does not exclude the presence of other elements. However, it is also to be understood that a description on a product/composition comprising certain components also discloses a product/composition consisting of these components. The product/composition consisting of these components may be advantageous in that it offers a simpler, more economical process for the preparation of the product/composition. Similarly, it is also to be understood that a description on a process comprising certain steps also discloses a process consisting of these steps. The process consisting of these steps may be advantageous in that it offers a simpler, more economical process.

When values are mentioned for a lower limit and an upper limit for a parameter, ranges made by the combinations of the values of the lower limit and the values of the upper limit are also understood to be disclosed.

The invention is now elucidated by way of the following examples, without however being limited thereto.

### Spin coating of a chitosan hydrogel on Si wafers.

Si wafers were cut by diamond cutter in 1 x 1 cm wafers, they were separately sonicated in ethanol in an ultrasonic bath for 10 min and washed twice with ethanol and MilliQ water. Wafers were dried under a stream of N₂ and treated with plasma for 20 min to remove any organic impurity and generate hydroxyl groups at the surface. The coating solution was prepared mixing 3.5 µl of 25 % v/v solution of glutaraldehyde to 1 mL of 0.35 % w/v solution of chitosan in 1 % acetic acid. The solution was mixed by vortex for 30 s and 50 µl were directly dispensed onto the plasma-treated Si wafer on the spin coater. The wafers were spun at 5000 RPM for 60 s with an acceleration time of 5 s. The chitosan coated wafers were stored in MilliQ water at room temperature for 24 h to desorb excess chitosan not physically bound to the surface. After 24 h they were carefully washed with fresh MilliQ water and dried under a stream of N₂ before the successive steps.

### Initiator attachment on chitosan film.

The chitosan coated wafers were immersed in 15 mL of a 0.24 M solution of triethylamine in THF. A 0.24 M solution of the initiator 2-bromo 2-methyl propionyl bromide in THF (7.5 mL) was added dropwise while the wafers were gently agitated. After 3 min of gentle agitation, the wafers were washed sequentially with THF, ethanol, MilliQ water and dried under a stream of N₂.

### Surface-initiated photo SET-LRP of poly(MeOEGMA) and poly(HEMA).

A stock solution was prepared dissolving CuBr₂ (3.9 mM) and the ligand Me₆TREN (23.4 mM) in DMSO. The stock solution was stirred at room temperature until complete dissolution, avoiding light exposure. The polymerization solution was prepared dissolving 7.92 mmol of the monomer and 200 µl of stock solution in 4.5 mL of DMSO in a round bottom flask wrapped in Al foil to prevent light exposure. The polymerization solution was degassed by bubbling with N₂ for 30 min. The wafers with the initiator immobilized on the chitosan layer were degassed separately with N₂ for 15 min before the addition of the polymerization solution. The polymerization reaction was performed in a UV reactor equipped with a λ 320 - 400 nm lamp. The polymerization was stopped at selected time points by opening the vials, diluting the polymerization solution with fresh DMSO and washing the wafers sequentially with ethanol and MilliQ water. The wafers were dried under N₂.

### Ellipsometry.

The dry thickness of the layers was measured using an OMT Ellipsometer and VisuEI software version 3.4.1 (Optische Messtechnik GmbH) at the angle of incidence of 70° and a spectral method with wavelength range from 460 to 870 nm.

### Assessment of the antifouling properties by SPR.

Non-specific protein adsorption (fouling) was measured on chitosan-coated surfaces before and after growth of the polymer layer *via* surface plasmon resonance (SPR) spectroscopy using an instrument based on spectral interrogation. Solutions were driven through a flow cell by a peristaltic pump at a flow rate of 25 µL·min⁻¹ and the SPR responses were measured as shifts in the resonant wavelength, λᵣₑₛ. Firstly, a baseline was established in phosphate buffered saline (PBS, pH 7.4). Solutions in PBS of Fbg (1 mg mL⁻¹) or HSA (5 mg mL⁻¹), HBP (10 % in PBS), or undiluted FBS were subsequently flowed over the surface for 15 min, after which the flowing solution was replaced with PBS. The sensor response (Δλres) was obtained as the difference between the baselines in PBS before and after the injection of the analyzed solutions and was converted to surface mass coverage using a calibration made previously (Δλres = 1 nm corresponds to an increase in the deposited protein mass of 15 ng cm-2.

### Hemocompatibility test.

The surfaces used for this study consisted of Si wafer coated with chitosan films by spin coating, either bare or functionalized with polymer brushes. To test the hemocompatibility of the poly(MeOEGMA) and poly(HEMA) brushes grafted from chitosan, the surfaces were incubated with platelet-rich plasma (PRP) and leukocytes isolated from fresh blood. Bare chitosan surfaces were used as a control. All individuals tested agreed to this study at the time of blood collection. All samples were obtained in accordance with the Ethical Committee regulations of the Institute of Hematology and Blood Transfusion, Prague and with release of informed consent. Platelets and leukocytes were obtained according to literature.

A 200 µl drop of PRP or leukocytes was deposited on the surfave to be tested and incubated for 60 min at 37 °C. Subsequently, the surfaces were thoroughly washed with 0.9% saline; fixed by glutaraldehyde (0.5%) for 60 min, rinsed with water, and then with ethanol and allowed to dry. The dried surfaes were sputter coated with 4 nm platinum, and observed using scanning electron microscopy (SEM). Images were evaluated using ImageJ data analysis software. The number of adherent platelets, and leukocytes was determined by calculating the surface coverage of individual components. Three independent spots were analyzed for each sample. The studies were carried out in triplicate.

### Chitosan films preparation and stability.

The films were homogeneous in thickness over the measured surface, with an average value of 12 nm.

Five of the coated wafers were stored in MilliQ water at room temperature for 52 d to check the stability of the layer. The thickness of the films was measured in dry state, after careful washing of the samples with fresh MilliQ water each time. There was little change in the thickness of the films, showing that the film is stable in solution up to two months,

### Photo-initiated SET-LRP of polymer brushes and surface characterization.

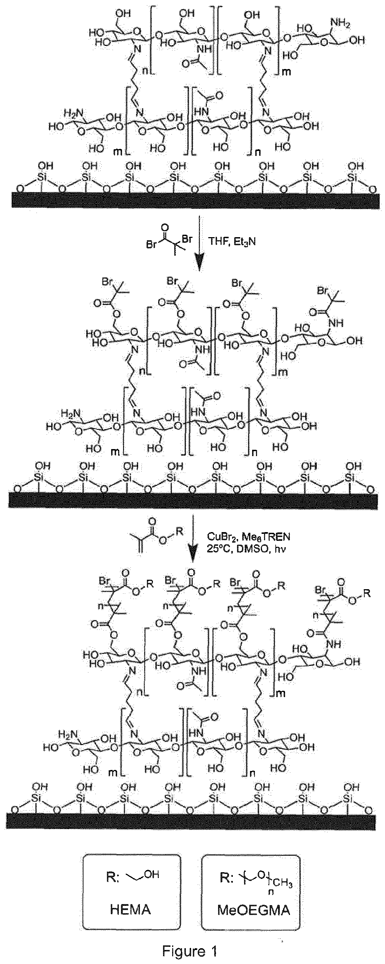

Essential to a graft-from-surface living radical polymerization is the presence of an alkyl halide initiator covalently attached to the surface. A common initiator group for SET-LRP consisting of 2-bromo-2-methylpropionate was used. The chitosan coated Si wafers were treated with a solution of the initiator in THF in basic conditions, an acylation reaction with the functional groups of chitosan ensures covalent linkage of the initiator to the hydrogel. The chitosan surfaces treated with 2-bromo 2-methylpropionyl bromide were used as the initiating site for grafting of poly(MeOEGMA) or poly(HEMA) by photo-initiated SET-LRP. This is shown in Figure 1.

Figure 1 shows an Illustration of the chitosan hydrogel deposited on top of Si wafers and stabilized by weak interactions between the chitosan monomers and the OH groups on the surface of the Si wafers. The initiator reacts by acylation with the free functional groups of the chitosan monomers.

Polymer brushes films with thickness of 180 nm and 50 nm were obtained respectively for HEMA and MeOEGMA, in less than 30 min. The chemical structure of the surfaces and the presence of different polymer brushes on the chitosan layers were confirmed by XPS.

It is worth noting that the XPS spectra of the surfaces modified with polymer brushes lack the spectral features of the chitosan layer. This confirms that the polymer layers are thick enough to cover the chitosan film and attenuate its signals (over 8 - 10 nm).

The overall uniformity observed within the phase AFM images confirms the homogeneous surface coverage, smoothness and absence of pinholes, which are necessary requirements to prevent protein adsorption. The polymerization procedure used enables the growth dense and uniform polymeric surfaces on top of a soft hydrogel base.

### Assessment of antifouling properties of polymer brushes.

Protein adsorption was investigated by surface plasmon resonance (SPR) on the prepared surfaces. Chitosan films were prepared on gold chips for SPR by spin-coating and they were functionalized with polymer brushes using the same synthetic procedures as on Si. The thickness of the chitosan layers was about 10 nm and for the polymer brush coatings a thickness of approx. 20 nm was selected based on previous results of fouling resistance on model surfaces. Four model solutions of fibrinogen (Fbg, 1 mg mL⁻¹), human serum albumin (HSA, 5 mg mL⁻¹), diluted human blood plasma (HBP, 10 % in PBS) and fetal bovine serum (FBS, undiluted) were put in contact with the prepared films.

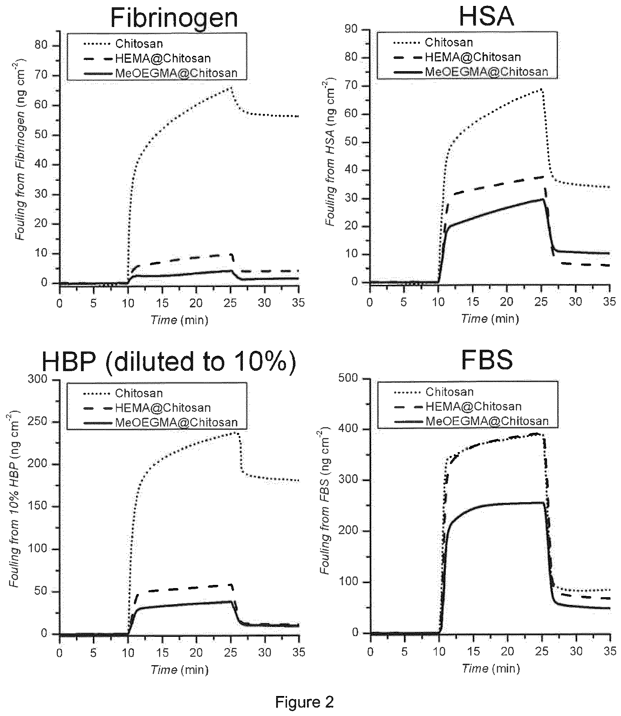

Importantly, bare chitosan suffers rapid adsorption of Fbg when contacted with a single-protein solution at a concentration similar to human plasma (Figure 2). This protein is important in the coagulation cascade, mediating the aggregation of activated platelets, and is negatively charged at physiological pH, thus it is attracted by the surface due to the weak cationic character of chitosan. Upon adsorption, intact Fbg molecules can under conformational changes revealing cryptic integrin binding sites for platelets and leukocytes. Thus, this protein also plays an early central role in the triggering of surface thrombogenicity and inflammation.

Importantly, both poly(HEMA) and poly(MeOEGMA) were able to reduce the fouling from Fbg by 93 % and 97 %, respectively. This represents a significant improvement in comparison with previously presented chitosan coating methods, owing to the dense brush architecture achieved *via* the grafting-from approach.

Moreover, the coating of chitosan with poly(HEMA) and poly(MeOEGMA) reduced the adsorption of HSA, the most abundant protein in plasma, by 83 % and 70 %, respectively. Nevertheless, resistance to the adsorption of individual proteins from simple solutions in buffer does not imply the ability to prevent fouling from complex biological fluids, which represent a much more demanding test.

For this purpose, fouling from HBP at 10 % in PBS was assessed. The antifouling properties of the polymer brush-coated chitosan layers were evidenced by the marked reduction in fouling from this challenging medium, showing a decrease of 94 % for poly(HEMA) and 95 % for poly(MeOEGMA) in comparison with bare chitosan, which experienced rapid protein fouling in this medium. In comparison FBS only showed a marginal enhancement of the antifouling behavior when compared to the bare chitosan solution, however it should be noted that the desorption of FBS from chitosan is already higher than for the other model solutions.

Figure 2 shows. surface plasmon resonance plots for chitosan, chitosan functionalized with poly(MeOEGMA) and chitosan functionalized with poly(HEMA) in contact with fibrinogen (1 mg mL⁻¹), human serum albumin (HSA, 5 mg mL⁻¹), diluted human blood plasma (HBP, 10% in PBS) and fetal bovine serum (FBS, undiluted).

Importantly, blood compatibility studies assessed the biocompatibility of the coating with respect to leukocytes and platelet adhesion. Leucocytes and platelets adhesion was fully prevented avoiding triggering of immune response and thrombogenicity.

## Claims

1. A coating comprising a hydrogel layer of a hydrogel of a polysaccharide and polymer brushes attached to the hydrogel layer.

2. The coating according to claim 1, wherein the polysaccharide is selected from the group consisting of alginate, chitosan, hyaluronan and chondroitin sulfate.

3. The coating according to any one of the preceding claims, wherein the polymer brushes comprise polymers of a monomer represented by formula (I) or (II) 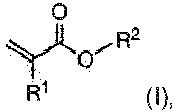 wherein R¹ is H, CH₃ or C₂H₅ and R² is selected from the group consisting of: C1-C12 linear, branched or cyclic alkyl,
- -R⁶-OH wherein R⁶ is C1-C12 linear, branched or cyclic alkyl,
- -R⁷-NH2 wherein R⁷ is C1-C12 linear, branched or cyclic alkyl; 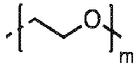 wherein m is 1 to 12, 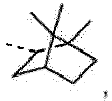 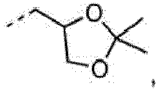 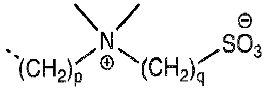 wherein p is 1 to 5 and q is 1 to 5 and 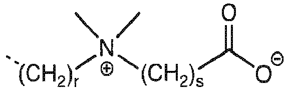 wherein r is 1 to 5 and s is 1 to 5; 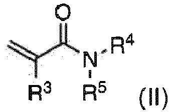
wherein R³ is H, CH₃ or C₂H₅ and
R⁴ and R⁵ are each independently selected from the group consisting of: H, C1-C12 linear, branched or cyclic alkyl;
- -R⁸-OH wherein R⁸ is C1-C12 linear, branched or cyclic alkyl,
- -R⁹-NH₂ wherein R⁹ is C1-C12 linear, branched or cyclic alkyl; 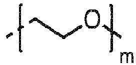 wherein m is 1 to 12, 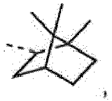 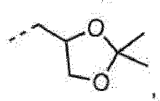 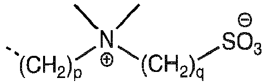 wherein p is 1 to 5 and q is 1 to 5 and 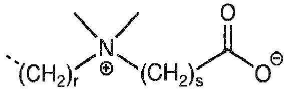 wherein r is 1 to 5 and s is 1 to 5.

4. The coating according to claim 3, wherein the monomer is selected from the group consisting of
2-(Dimethylamino)ethyl methacrylate (DMAEMA),
2-hydroxyethyl methacrylate (HEMA),
2-hydroxypropyl methacrylate (HPMA),
oligo(ethylene glycol) methyl ether methacrylate (MeOEGMA),
t-butyl methacrylate (tBMA),
n-butyl methacrylate (nBMA),
methyl methacrylate (MMA),
2-ethylhexyl methacrylate (EHMA),
isobornyl methacrylate (IBMA),
solketal methacrylate (SMA), [2-(methacryloyloxy)ethyl]dimethyl-(3-sulfopropyl)ammonium hydroxide (SBMA) 2-Carboxy- N,N-dimethyl-N-(2'-(methacryloyloxy)ethyl)ethanaminium (CBMA).

5. The coating according to any one of the preceding claims, wherein the hydrogel layer has a thickness of 3-20 nm as measured by ellipsometry in a dry state.

6. The coating according to any one of the preceding claims, wherein the polymer brushes form a layer having a thickness of 10-500 nm as measured by ellipsometry in a dry state.

7. A process for making the coating according to any one of the preceding claims, comprising the steps of:
a) providing the hydrogel layer on a substrate,
b) attaching an initiator having a radically transferable atom on the hydrogel layer and
c) reacting monomers with the initiator to form the polymer brushes attached to the hydrogel layer.

8. The process according to claim 7, wherein the substrate is selected from the group consisting of silicon, glass, stainless steel, platinum. platinum iridium, nitinol, tantalum, titanium, cobalt-chromium alloy, copper, silver, copper-silver alloy, silicone, polyethylene, polycarbonate, polyurethane, polycaprolactone, silk and collagen.

9. The process according to claim 7 or 8, wherein step a) involves crosslinking the polysaccharide on the substrate to form the hydrogel layer.

10. The process according to any one of claims 7-9, wherein step c) is performed by living free-radical polymerization.

11. The process according to any one of claims 7-10, wherein step c) is performed by SET-LRP.

12. The process according to any one of claims 7-11, wherein the initiator is represented by 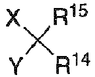 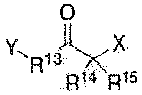 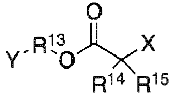 or 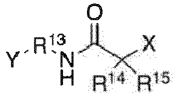 wherein
X is selected from the group consisting of Cl, Br and I;
Y is an anchoring group which reacts with functional groups of the polysaccharide;
R¹³ is selected from the group consisting of C₁-C₂₀ linear or branched alkyl, C₂-C₆ alkenyl, C₂-C₆ alkynyl, N-hydroxysuccinimide, poly(ethylene glycol) methyl ether, C₁-C₂₀ alkyl substituted with e.g. an azide, and 2,2-dimethyl-1,3-dioxolan-4-yl and
R¹⁴ and R¹⁵ are each independently selected from the group consisting of C₁-C₄ linear alkyl, C₂-C₈ cycloalkyl, aryl, heterocyclyl, aralkyl, aralkenyl.

13. A medical device provided with, preferably encapsulated in, the coating according to any one of claims 1-6.

14. The medical device according to claim 13, wherein the medical device is selected from the group consisting of an implantable biosensor, a feeding tube, a central line catheter, a nephrostemy catheter, a guiding catheter, a pacemaker, an internal insulin pump, an orthopedic hip implant, an orthopedic screw, a dental implant, a percutaneous fixation device, a peripherally inserted central catheter, a drainage catheter and a stent such as coronary stent, vascular stent, ureteral stent, prostatic tent, esophageal stent and biliary stent.

15. Use of the coating according to any one of claims 1-6 for encapsulating a medical device, in particular an implantable biosensor.
